# EUROPEAN PATENT SPECIFICATION

(11) **EP 2 756 299 B1**
(45) Date of publication and mention of the grant of the patent: **09.11.2016**
(21) Application number: 12758488.6
(22) Date of filing: 13.09.2012
(51) Int. Cl.: G01N 33/50, G01N 33/569

(54) **AN IN-VITRO PROCESS FOR THE QUICK DETERMINATION OF THE INFECTION STATUS OF AN INFECTION WITH AN INFLUENZA VIRUS TYPE**
IN-VITRO-VERFAHREN FÜR DIE SCHNELLE BESTIMMUNG EINES INFEKTIONSZUSTANDS EINER INFEKTION MIT EINEM INFLUENZA-VIRUSTYP
PROCÉDÉ IN VITRO POUR LA DÉTERMINATION RAPIDE DU STATUT INFECTIEUX D'UNE INFECTION PAR UN TYPE DE VIRUS DE LA GRIPPE

(30) Priority: 13.09.2011 EP 11181119; 13.09.2011 US 201161534058 P
(43) Date of publication of application: 23.07.2014
(73) Proprietor: Sovicell Gmbh, 04103 Leipzig (DE)
(72) Inventor: BAHLMANN, Ferdinand Hermann, 66121 Saarbrücken (DE); FLISER, Danilo, 69120 Heidelberg (DE); SESTER, Martina, 66424 Homburg (DE); SESTER, Urban, 66424 Homburg (DE)
(74) Representative: dompatent von Kreisler Selting Werner - Partnerschaft von Patent- und Rechtsanwälten mbB
(86) International application number: PCT/EP2012/067928
(87) International publication number: WO 2013/037878

(56) References cited:
- WO-A2-2007/107714
- URBAN SESTER ET AL: "Whole-Blood Flow-Cytometric Analysis of Antigen-Specific CD4 T-Cell Cytokine Profiles Distinguishes Active Tuberculosis from Non-Active States", PLOS ONE, PUBLIC LIBRARY OF SCIENCE, vol. 6, no. 3, 15 March 2011 (2011-03-15), pages E17813-1, XP002631779, ISSN: 1932-6203, DOI: 10.1371/JOURNAL.PONE.0017813
- G. PEDERSEN ET AL: "Pandemic Influenza Vaccination Elicits Influenza-Specific CD4+ Th1-cell Responses in Hypogammaglobulinaemic Patients: Four case reports", SCANDINAVIAN JOURNAL OF IMMUNOLOGY, vol. 74, no. 2, 6 July 2011 (2011-07-06), pages 210-218, XP055022455, ISSN: 0300-9475, DOI: 10.1111/j.1365-3083.2011.02561.x
- BROWN DEBORAH M ET AL: "CD4 T cell responses to influenza infection.", SEMINARS IN IMMUNOLOGY JUN 2004 LNKD- PUBMED:15130501, vol. 16, no. 3, June 2004 (2004-06), pages 171-177, XP002672020, ISSN: 1044-5323

## Description

The present invention pertains to an in-vitro process for the quick determination of the infection status of an infection with an influenza virus type from whole blood in terms of an acute influenza infection or a lasting-immunization due to an previous influenza infection or vaccination.

Influenza, commonly referred to as the flu, is an infectious disease caused by the influenza viruses, that affects birds and mammals. The most common symptoms of the disease are chills, fever, sore throat, muscle pains, severe headache, coughing, weakness/fatigue and general discomfort.

New influenza viruses are constantly evolving by mutation or by reassortment. Mutations can cause small changes in the hemagglutinin and neuraminidase antigens on the surface of the virus. This is called antigenic drift, which slowly creates an increasing variety of strains until one evolves that can infect people who are immune to the pre-existing strains. This new variant then replaces the older strains as it rapidly sweeps through the human population-often causing an epidemic. However, since the strains produced by drift will still be reasonably similar to the older strains, some people will still be immune to them. In contrast, when influenza viruses reassort, they acquire completely new antigens-for example by reassortment between avian strains and human strains; this is called antigenic shift. If a human influenza virus is produced that has entirely new antigens, everybody will be susceptible, and the novel influenza will spread uncontrollably, causing a pandemic.

Typically, influenza is transmitted through the air by coughs or sneezes, creating aerosols containing the virus. Influenza can also be transmitted by direct contact with bird droppings or nasal secretions, or through contact with contaminated surfaces. Airborne aerosols have been thought to cause most infections, although which means of transmission is most important is not absolutely clear.

As influenza is caused by a variety of species and strains of viruses, in any given year some strains can die out while others create epidemics, while yet another strain can cause a pandemic. Typically, in a year's normal two flu seasons (one per hemisphere), there are between three and five million cases of severe illness and up to 500,000 deaths worldwide, which by some definitions is a yearly influenza epidemic.

Three influenza pandemics occurred in the 20th century and killed tens of millions of people, with each of these pandemics being caused by the appearance of a new strain of the virus in humans. Often, these new strains appear when an existing flu virus spreads to humans from other animal species, or when an existing human strain picks up new genes from a virus that usually infects birds or pigs. An avian strain named H5N1 raised the concern of a new influenza pandemic, after it emerged in Asia in the 1990s, but it has not evolved to a form that spreads easily between people. In April 2009 a novel flu strain evolved that combined genes from human, pig, and bird flu, initially dubbed "swine flu" and also known as influenza A/H1N1, emerged in Mexico, the United States, and several other nations. The World Health Organization officially declared the outbreak to be a pandemic on June 11, 2009.

In virus classification influenza viruses are RNA viruses that make up three of the five genera of the family Orthomyxoviridae: Influenza virus A, B, and C.

### Influenzavirus A

Wild aquatic birds are the natural hosts for a large variety of influenza A. Occasionally, viruses are transmitted to other species and may then cause devastating outbreaks in domestic poultry or give rise to human influenza pandemics. The type A viruses are the most virulent human pathogens among the three influenza types and cause the most severe disease. The influenza A virus can be subdivided into different serotypes based on the antibody response to these viruses. The serotypes that have been confirmed in humans, ordered by the number of known human pandemic deaths, are:
▪ H1N1, which caused Spanish Flu in 1918, and Swine Flu in 2009
▪ H2N2, which caused Asian Flu in 1957
▪ H3N2, which caused Hong Kong Flu in 1968
▪ H5N1, which caused Bird Flu in 2004
▪ H7N7, which has unusual zoonotic potential[23]
▪ H1N2, endemic in humans, pigs and birds
▪ H9N2
▪ H7N2
▪ H7N3
▪ H10N7

### Influenzavirus B

Influenza B almost exclusively infects humans and is less common than influenza A. The only other animals known to be susceptible to influenza B infection are the seal and the ferret. This type of influenza mutates at a rate 2-3 times slower than type A and consequently is less genetically diverse, with only one influenza B serotype. This reduced rate of antigenic change, combined with its limited host range (inhibiting cross species antigenic shift), ensures that pandemics of influenza B do not occur.

### Influenzavirus C

Influenzavirus C infects humans, dogs and pigs, sometimes causing both severe illness and local epidemics. However, influenza C is less common than the other types and usually only causes mild disease in children.

As prophylaxis against an influenza A and B virus infections vaccination is possible. Influenza virus is frequently changing its structures which are recognized by the immune system. Various types of influenza virus can be active at the same time e.g. influenza viruses which are responsible for the "normal" seasonal influenza diseases for example H3N2 and more dangerous ones for example H5N1 (bird's flue). Vaccinations against influenza are usually made available to people in developed countries. Farmed poultry is often vaccinated to avoid decimation of the flocks. The most common human vaccine is the trivalent influenza vaccine (TIV) that contains purified and inactivated antigens against three viral strains. Typically, this vaccine includes material from two influenza A virus subtypes and one influenza B virus strain. The TIV carries no risk of transmitting the disease, and it has very low reactivity. A vaccine formulated for one year may be ineffective in the following year, since the influenza virus evolves rapidly, and new strains quickly replace the older ones. Antiviral drugs can be used to treat influenza, with neuraminidase inhibitors (such as Tamiflu or Relenza) being particularly effective.

H3N2 and more dangerous ones for example H5N1 (bird's flue). Vaccinations against influenza are usually made available to people in developed countries. Farmed poultry is often vaccinated to avoid decimation of the flocks. The most common human vaccine is the trivalent influenza vaccine (TIV) that contains purified and inactivated antigens against three viral strains. Typically, this vaccine includes material from two influenza A virus subtypes and one influenza B virus strain. The TIV carries no risk of transmitting the disease, and it has very low reactivity. A vaccine formulated for one year may be ineffective in the following year, since the influenza virus evolves rapidly, and new strains quickly replace the older ones. Antiviral drugs can be used to treat influenza, with neuraminidase inhibitors (such as Tamiflu or Relenza) being particularly effective.

WO 2007/107714 A2 discolses a method of assessing an intracellular pathogen infection and/or monitoring an intracellular pathogen infection in an individual comprising the determination whether the individual has (a) T-cells that secrete IFN-γ only, (b) T-cells that secrete IL-2 only or (c) T-cells that secrete both IFN- y and IL-2 in response to an intracellular pathogen antigen and optionally determining any change in this cytokine profile. The pathogens described in Detail are CMV, hepatitis virus, EBV or HSV. These viruses do not rapidly change its antigenic pattern like influenza viruses, so that the method known from WO 2007/107714 A2 cannot readily adopted to solve the problem underlying the invention.

Urban Sester et al., in PLoS one, vol. 6, no. 3, pages E17813 disclose that T-cell based IFN-γ release assays do not permit distinction of active tuberculosis (TB) from successfully treated disease or latent *M. tuberculosis* infection. The authors postulated that IFN-γ and IL-2 cytokine profiles of antigen-specific T cells measured by flow-cytometry *ex vivo* might correlate with TB disease activity *in vivo*. Tuberculin (PPD), ESAT-6 and CFP-10 were used as stimuli to determine antigen-specific cytokine profiles in CD4 T cells from 24 patients with active TB and 28 patients with successfully treated TB using flow-cytometry. Moreover, 25 individuals with immunity consistent with latent *M. tuberculosis* infection and BCG-vaccination, respectively, were recruited. Although the frequency of cytokine secreting PPD reactive CD4 T cells was higher in patients with active TB compared to patients with treated TB (median 0.81% vs. 0.39% of CD4 T cells, p = 0.02), the overlap in frequencies precluded distinction between the groups on an individual basis. When assessing cytokine profiles, PPD specific CD4 T cells secreting both IFN-γ and IL-2 predominated in treated TB, latent infection and BCG-vaccination, whilst in active TB the cytokine profile was shifted towards cells secreting IFN-γ only (p<0.0001). Cytokine profiles of ESAT-6 or CFP-10 reactive CD4 T cells did not differ between the groups. Receiver operator characteristics (ROC) analysis revealed that frequencies of PPD specific IFN-γ/IL-2 dual-positive T cells below 56% were an accurate marker for active TB (specificity 100%, sensitivity 70%) enabling effective discrimination from non-active states. In conclusion, a frequency lower than 56% IFN-γ/IL-2 dual positive PPD-specific circulating CD4 T-cells is strongly indicative of active TB.

Prior to vaccination it is desirable to know whether a patient is not yet infected, is at present suffering from an active influenza and if so whether the patient is infected by "normal" influenza viruses or e.g. H5N1.

The invention is based on the observation that T cellular immune responses against different Influenza strains could be detected and characterized with the aid of antigen specific stimulation of T cells within whole blood samples. Influenza reactive T cells could be detected after leukocyte fixation and lysis of red blood cells with the aid of intracellular cytokine staining and subsequent analysis of their frequency among all respective T cells by flow cytometry. It was observed in some individuals a low level pre existing influenza specific T cells and in all individuals a distinct increase of influenza specific T cells after vaccination. Interestingly this increase was accompanied by a change in the cytokine profile of the activated T cells which was most dominant one week after vaccination. In some other unvaccinated individuals, which presented with clinical signs suggestive for acute influenza infection, the analysis of influenza specific T cells was performed to confirm a newly induced immune response. In these individuals, there was an even more striking change in cytokine profile of activated T cells. The in-vitro process of the invention allows for the determination of an actual infection status of an individual.
- Fig. 1:: Immunisation with the pandemic influenza vaccine induces a specific CD4 T-cell response towards both pandemic and seasonal influenza antigens.
- Fig. 2:: Vaccination mainly induces Th1-cells of an effector memory phenotype.
- Fig. 3:: A pre-existing immunity prevents strong induction of antigen specific CD4 T cells.
- Fig. 4:: Vaccine-induced antibodies show only minor cross-reactivity with seasonal antigens.
- Fig. 5:: Differences in the cytokine expression profile of pdmH1N1 - specific CD4 T cells discriminate memory or vaccination responses from acute infection.

The process of the invention is an in-vitro process for the quick determination of the infection status of an infection with an influenza virus type from whole blood of humans and animals in terms of an acute influenza infection, and induced immune response due to vaccination or a pre-immunization due to previous, overcome influenza infection, comprising the steps of:
- stimulating an antigen-specific T cell that is present in a first sample of whole blood with antigens of the influenza virus type, optionally in the presence of antibodies against CD28, or CD28 and CD49d;
- processing of the influenza antigens by antigen-presenting cells (APC) by incubation for a sufficient time, in particular by incubation of at least 0,5 h, particularly of 1.5 h to 2.5 h, especially for 2 h, at 35 °C to 39 °C, especially at 37 °C, optionally with adding CO₂;
- then adding a secretion inhibitor;
- effecting an intensive mixing;
- followed by a second incubation step for a period of at least 1 h, in particular 2,5 h, particularly of 3.5 h to 6 h, especially for 4 h, at 35 °C to 39 °C, especially at 37 °C, optionally with adding CO₂;
- determining a cytokine profile from both the intracellular INF-γ production and the intracellular IL-2 production of the antigen-specific T cell; wherein
- the presence of an acute influenza infection is indicated by a shift of the profile towards IFN-γ single positive cells accompanied by a ratio of IFN-y/IL-2 single positive T cells larger than 1 compared to the ratio of IFN-y/IL-2 single positive T cells of patients successfully vaccinated against, pre-infected or non-infected by the influenza virus which is smaller than 1.

According to the invention a negative control can be performed by adding a physiological buffer such as PBS 0.9% NaCl or 5% Glucose to an antigen-specific T cell that is present in the whole blood of a second sample, which is authentic with said first sample.

A positive control can be performed by adding SEB to an antigen-specific T cell that is present in the whole blood of a third sample, which is authentic with said first sample.

In still another embodiment of the invention a vaccination control can take place by adding the vaccination antigen to an antigen-specific T-cell that is present in the whole blood of a fourth sample which is authentic with said first sample.
In the process according to the invention the secretion inhibitor is selected from the group consisting of brefeldin A, monensin or the like.

The determination of the cytokine profile can be effected by flow cytometry, automated microscopy or multiplex elisa methods.

The invention is further illustrated by way of the following more specific examples and descriptions which are not intended to limit the scope of the claims. Induction of antigen-specific antibody responses is well-characterised after vaccination with pandemic H1N1 or seasonal influenza (Flu) vaccines. However, the kinetics of cellular immune responses towards pandemic H1N1 and cross-reactivities towards seasonal Flu vaccine in respect to changes in cytokine profiles of antigen specific T cells are not known so far.

19 immunocompetent individuals were vaccinated with the pandemic H1N1 vaccine Pandemrix and CD4-T-cell frequencies specific for pandemic H1N1 and for seasonal Flu vaccine were monitored before and 1, 2 and 10 weeks after vaccination using intracellular cytokine-staining (IFN-γ, IL-2, IL-17). Stimulation with staphylococcus aureus enterotoxin B (SEB) served as positive control. Apart from cytokine analyses, specific T-cells were phenotypically characterised by memory and maturation markers. Moreover, induction of specific antibodies was monitored by ELISA and neutralisation assay. In addition, 7 patients with acute H1N1 infection were analysed.

Prior to vaccination, levels of pandemic H1N1-specific CD4-T-cells were below detection limit in 68% of cases. In contrast, immunity towards seasonal Flu was detectable in 79%. Specific T-cell responses after vaccination rose to above detection limit in all cases. Median frequencies increased by 463% (IQR 291-715%) and remained stable throughout follow-up. Interestingly, median T-cell levels towards seasonal vaccine concomitantly rose from 0.10% to 0.24% whereas SEB-reactive T-cells were not specifically induced after vaccination. Influenza-specific cells before and after vaccination were co-expressing IFN-γ and IL-2, whereas IL-17 was not detectable. In contrast, T cells in patients with active H1N1 infection predominantly expressed IFN-γ only. In contrast to the cellular immunity, only H1N1-pandemic antibody responses but no cross-reactive antibodies against the seasonal Flu vaccine were induced.

In conclusion, besides specific induction of H1N1-reactive T-cell immunity, pandemic H1N1 vaccination also induces a cross-protective T-cell response towards seasonal influenza vaccines. In addition, cytokine-profiling may be applied as diagnostic measure to identify patients with active H1N1 infection.

### Study subjects

The vaccination study was conducted among 19 immunocompetent individuals in October/November 2009. Among them, 10 persons (mean age 43.64±10.05 years) had been pre-vaccinated against seasonal influenza (Begrivac 2009/2010, Novartis, Marburg, Germany) about 3 weeks before (mean 18.9±7.9 days), whereas 9 (mean age 37.8±15.9 years) had not. All individuals received one standard dose of the pdmH1N1 vaccine (Pandemrix, GlaxoSmithKline Biologicals s.a., Rixensart, Belgium), and all gave informed consent. Whole blood samples were drawn prior to vaccination with Pandemrix as well as 1, 2, and 10 weeks thereafter. To characterize cellular immunity in acute infection, additional whole blood samples of 7 individuals (mean age 49.28±11.07 years) who were newly diagnosed with influenza A/H1N1 infection on a clinical/epidemiological or virological basis were analyzed.

### Quantitation and phenotypic analysis of influenza-specific CD4 T cells

T cells from heparinized whole blood sample were stimulated *in vitro* for 6 h as described previously [1]. A total of 450µl blood was used per stimulus. PdmH1N1 antigen (pdmH1N1, 30µg/ml HA content, derived from A/california(H1N1) was used in the pandemic vaccine Pandemrix) kindly provided by GlaxoSmithKline Biologicals s.a., Rixensart, Belgium and the seasonal influenza 2009/2010 vaccine Begrivac (Flu09, 30µg/ml HA content; Novartis, Nürnberg, Germany) were used as stimuli in the presence of 1µg/ml anti-CD28 and anti-CD49d (clones L293 and 9F10; BD, Heidelberg, Germany), respectively, to induce antigen-specific activation and cytokine expression. As negative and positive controls, respectively, cells were stimulated with phosphate buffered saline (PBS), and 2.5µg/ml *Staphylococcus aureus* Enterotoxin B (SEB, Sigma, Deisenhofen, Germany). Stimulated cells were fixed and immunostained using titered amounts of anti-CD4 (clone SK3), anti-CD69 (clone L78), anti-IFN-γ (clone 4S.B3), anti-IL-2 (clone MQ1-17H12), anti-IL-17A (clone SCPL1362), anti-CD152 (CTLA-4, clone BNI3; all from BD) and anti-CD127 (clone eBioRDR5, eBioscience-NatuTec, Frankfurt, Germany). At least 10.000 CD4 T-cells were analyzed on a FACS Canto II (BD) using FACSdiva software V 6.1.3.. For analyses of antigen-specific CD4 T cells, frequencies of activated CD4 T cells after stimulation with the antigen were subtracted for the respective negative controls. The detection limit was 0.05% IFN-γ-producing CD69+ CD4 T cells.

### Quantitation of influenza-specific antibody responses

Kinetics of pdmH1N1-specific IgG induction was analyzed using a standard ELISA (Virotech, Rüsselsheim, Germany), according to the manufacturer's instructions. Moreover, the presence of virus-neutralizing antibodies against pdmH1N1 and against the individual components of the seasonal influenza vaccine (H1N1seasonal, H3N2, Influenza B) in serum samples of subjects not pre-vaccinated with Begrivac was determined by respective microneutralization assays based on previously described procedures [2, 3]. In detail, heat inactivated (30min, 56°C) sera were used to prepare two-fold serial dilutions in duplicate from 1:10 to 1:1280 in virus diluent [EMEM (Biochrom, Germany) + 0,1% antibiotics + 2% FBS (Biochrom, Germany)]. The diluted sera were mixed with an equal volume (100 µl) of virus diluent containing influenza A virus [NYMC X-179A (H1N1) derived from A/California/7/2009] at 2x10³ TCID₅₀/ml, incubated for 90min (37°C, 5% CO₂) in a moist chamber and 100µl were transferred to MDCK cell monolayers (ECCC, Salisbury, UK) in a 96-well plate. Eight control wells containing influenza virus in virus diluent (virus control) or virus diluent alone (cell control) were included on each plate. After 1h incubation, 100 µl of overlay medium [1:1 virus diluent and 3.2% carboxy-methylcellulose (CMC, Sigma-Aldrich)] was added to each well for 24 to 28h. Then, the monolayers were fixed with ice cold acetone:methanol (40%:60%; 10min, RT). After blocking with 1% bovine serum albumin (Merck, Germany), 0.1% thimerosal (Sigma-Aldrich) in Dulbecco A wash buffer (8.0 g NaCl, 2.9 g Na₂HPO₄ 0.2 g KCl and 0.2 g K₂HPO₄ dissolved in 1000 ml of deionized water, pH 7.3) for 30 min at 37°C, 5% CO₂), fixed cells were subsequently incubated with 25µl anti-influenza Monoclonal antibodies to nucleoprotein (MAB8251, Millipore), diluted to 1:10.000 in blocking buffer. and 25 µl/well horseradish peroxidase-labeled rabbit anti-mouse IgG (P0161, Dako-Cytomation, Denmark) diluted 1:1000 in blocking buffer for 30 min at 37°C, 5% CO₂ respectively and substrate reaction was performed for 30min using 0.03% H₂O₂ in 50µl/well freshly prepared 3-amino-9-ethylcarbazole (Sigma-Aldrich) dissolved in 4.0 ml N,N-dimethylformamide (Sigma-Aldrich) and 16ml 0.05 M acetate buffer (pH 4.95-5.05). Immunostained foci were counted and recorded using automated ELISPOT instrumentation (AID Diagnostika GmbH, Straßberg, Germany). The neutralizing-antibody titer was expressed as the reciprocal of the highest dilution that reduced the number of immunostained foci to 50% or less of the control value.

### Results

Immunisation with the pandemic influenza vaccine induces a specific CD4 T-cell response towards both the pandemic and seasonal influenza-antigens.

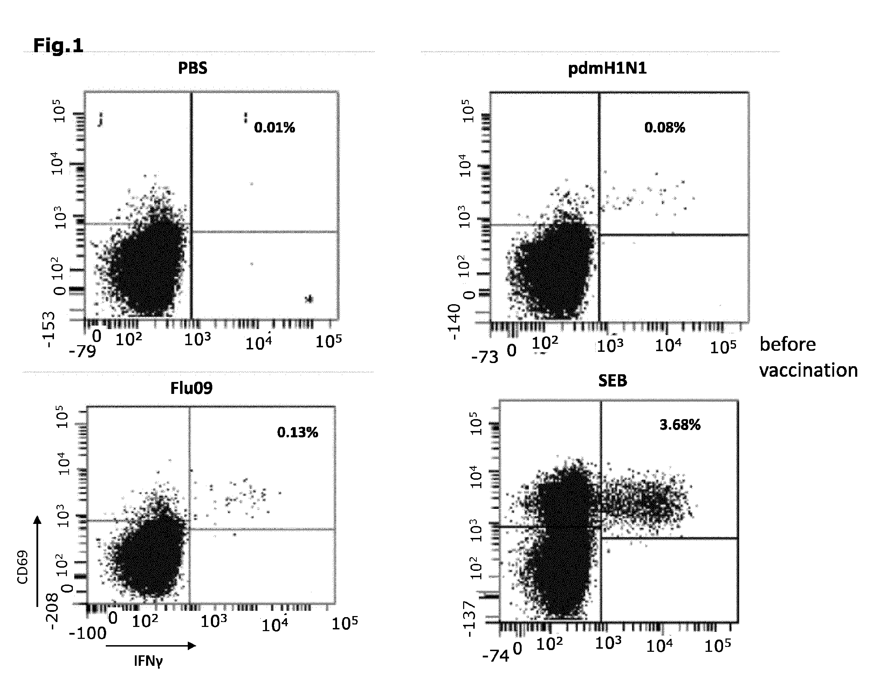

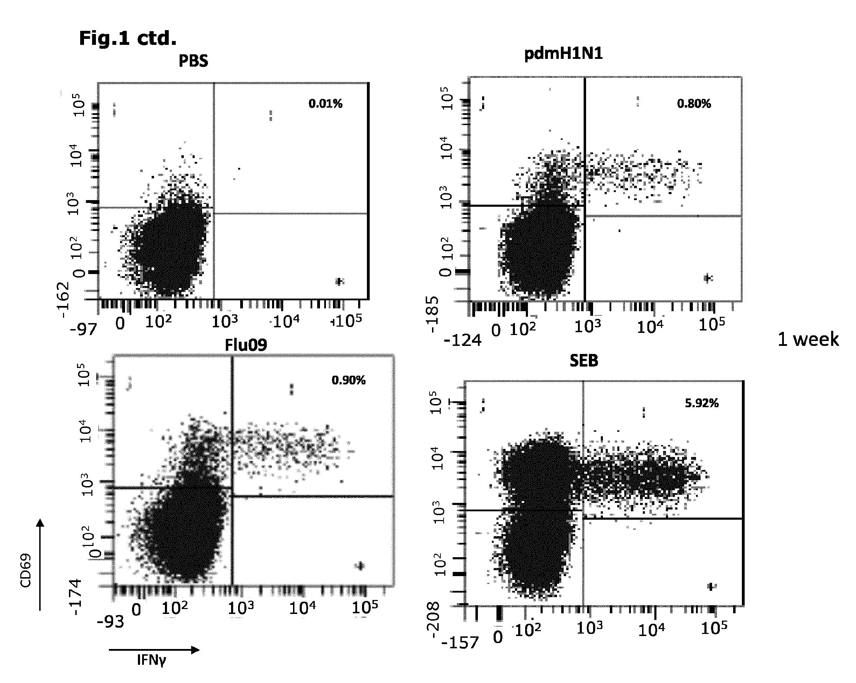

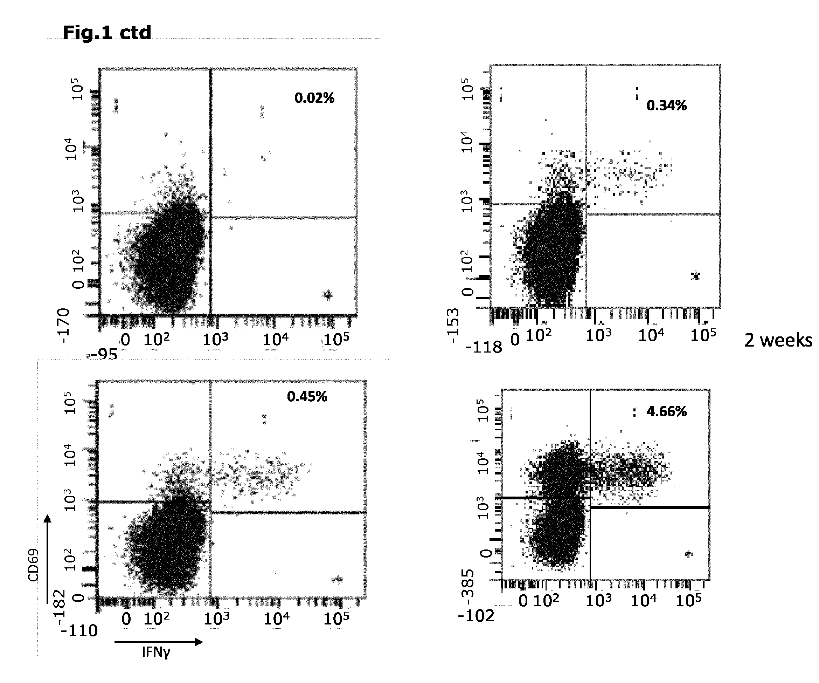

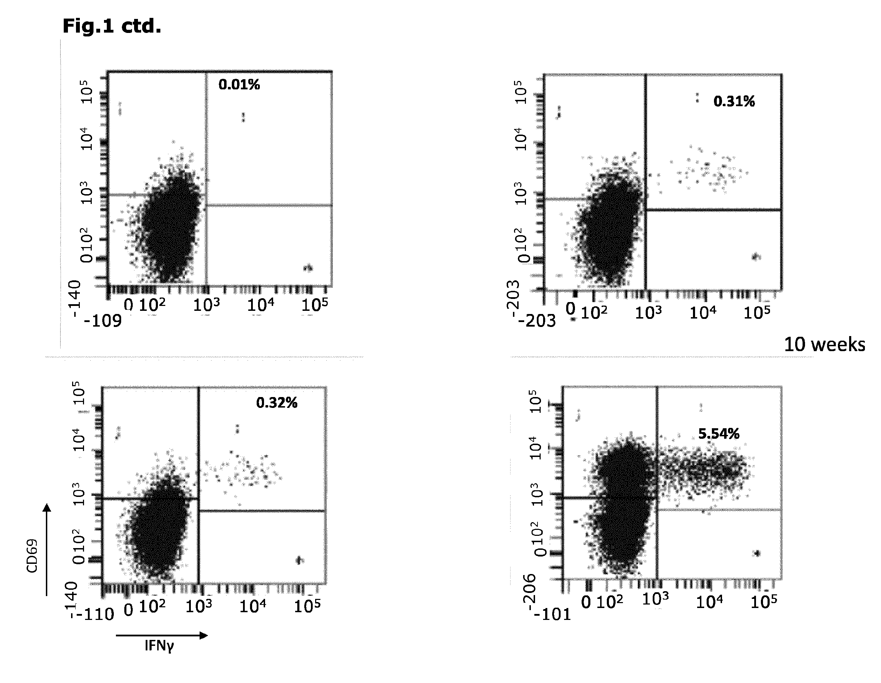

The induction of influenza-specific cellular immunity was assessed in a total of 19 individuals before as well as one, two and 10 weeks after immunisation with the pandemic vaccine Pandemrix. Among those, 10 individuals had been pre-vaccinated using the seasonal influenza vaccine. The frequencies of CD4 T cells specific for both the pandemic (pdmH1N1) and cross-reactive seasonal influenza-antigens (Flu09) were determined directly ex vivo from whole blood samples. Antigen-specific CD4 T cells were identified using flow-cytometry based on the induction of the activation marker CD69 and the cytokine IFN-γ after specific stimulation *in vitro*. When basal T-cell frequencies in all 19 individuals were assessed, low levels of CD4 T cells specific for pdmH1N1 (median (IQR) 0.03% (0.04%)) and seasonal influenza antigens were found (0.10% (0.07%), table 1). Figure 1: Immunisation with the pandemic influenza vaccine induces a specific CD4 T-cell response towards both pandemic and seasonal influenza antigens. Whole blood samples taken prior to as well as one, two and 10 weeks after immunisation with the pandemic influenza vaccine were stimulated with phosphate buffered saline (PBS, negative control), the pandemic H1N1 antigen (pdmH1N1), the 2009 seasonal influenza vaccine Begrivac (Flu09) and the *Staphylococcus aureus* enterotoxin B (SEB, positive control), respectively, and reactive CD4 T cells (CD69+/IFN-γ+) were counted using flow cytometry. Typical dotplots of one representative individual that was not pre-vaccinated with the seasonal vaccine are shown. The numbers indicate the percentage of antigen-reactive CD4 T cells. As shown in a representative example (Fig. 1), low levels of specific T cells present prior to vaccination increased thereafter and peak values were found 1 week after vaccination (Fig. 1 and table 1). In general, Pandemrix vaccination resulted in the strongest induction of specific CD4-T-cell frequencies towards the pdmH1N1 antigen present in the vaccine (4.63-fold median increase after one week, table 1). Interestingly, although less pronounced, this was associated with a concomitant increase in the percentage of cells cross-reacting towards seasonal influenza antigens (2.71-fold median increase). In contrast, frequencies of T cells reactive against the positive control stimulus SEB did not change over time (Fig. 1 and table 1). These results show that vaccination with the pandemic influenza vaccine Pandemrix induces a specific CD4 T-cell immunity towards pandemic H1N1 antigens that cross-reacts with those of the seasonal influenza vaccine. Vaccination mainly induces Th1-cells of an effector memory phenotype.

In order to analyse changes in phenotypical properties of vaccine-induced pdmH1N1-specific CD4 T cells in more detail, we monitored the expression of different functional markers (IFN-γ, IL-2, IL-17, CD69, CTLA-4) and of the memory marker CD127.

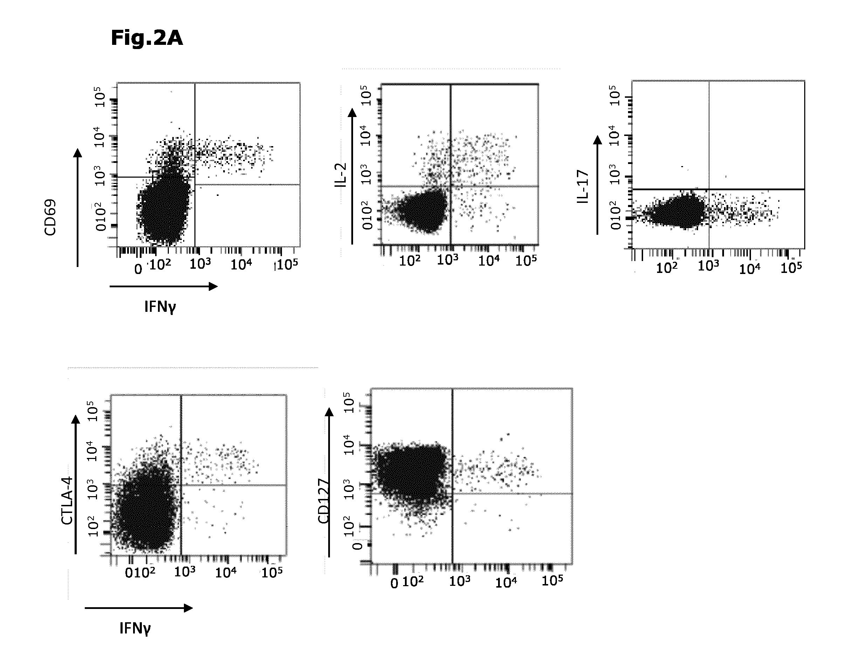

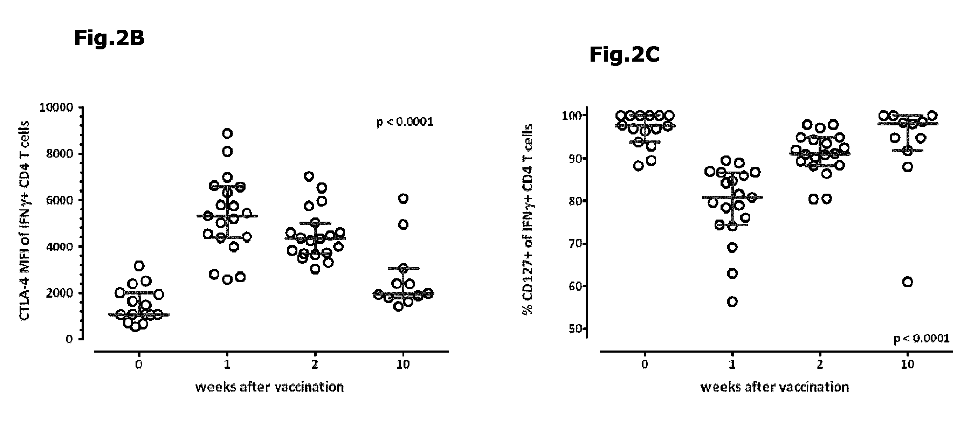

Figure 2: Vaccination mainly induces Th1-cells of an effector memory phenotype. Phenotypical characterisation of pdmH1N1-specific CD4 T cells after vaccination with Pandemrix. (A) Flow cytometric analysis of IFN-γ and CD69, IL-2, IL-17, CTLA-4 and CD127 expression of CD4 T cells stimulated with pdmH1N1 antigen in blood samples of a representative individual (not pre-vaccinated against seasonal influenza) one week after vaccination. (B) Mean fluorescence intensity (MFI) of the CTLA-4-signal of all IFN-γ+ CD4 T cells and (C) percentage of CD127-expressing cells of all IFN-γ+ CD4 T cells after stimulation with the pdmH1N1 antigen of all tested individuals are depicted over time (n=10 in week 10). Bars indicate median values and IQR.

As shown in representative dotplots of pdmH1N1-specific T cells one week after Pandemrix vaccination, predominant co-expression of effector cytokines IL-2 and IFN-γ with CD69 indicated that specific T cells were mainly Th1 effector-type cells, whereas no IL-17-expressing cells were detected (Fig. 2A). In line with the kinetics in frequency, CTLA-4, the most important negative co-stimulatory surface receptor of T-cell activation, peaked one week after Pandemrix vaccination and then decreased to almost pre-vaccination levels (p<0.0001, Fig. 2B). On the other hand, median (IQR) percentages of CD127-expressing influenza-specific CD4 T cells initially decreased from 97.6% (5.0%) to 80.0% (12.1%) during the first week post vaccination, but increased again in the follow-up to 98.0% (6.0%) after 10 weeks indicating activation of naive T cells followed by differentiation into effector memory cells (p<0.0001, Fig. 2C). The CD4 T cells that were specific for the seasonal influenza vaccine showed similar expression profiles and properties (data not shown). In summary, phenotypical characterisation of the influenza-specific CD4 T cells showed that the vaccine leads to induction of mainly Th1 effector memory cells that show dynamic changes in the immediate post-vaccination period.

A pre-existing immunity prevents strong induction of antigen specific CD4 T cells To assess the impact of pre-existing cellular immunity against seasonal influenza antigens on basal levels and on the induction of influenza specific immunity after Pandemrix vaccination, individuals were separated into those not pre-vaccinated and pre-vaccinated with the seasonal influenza vaccine.

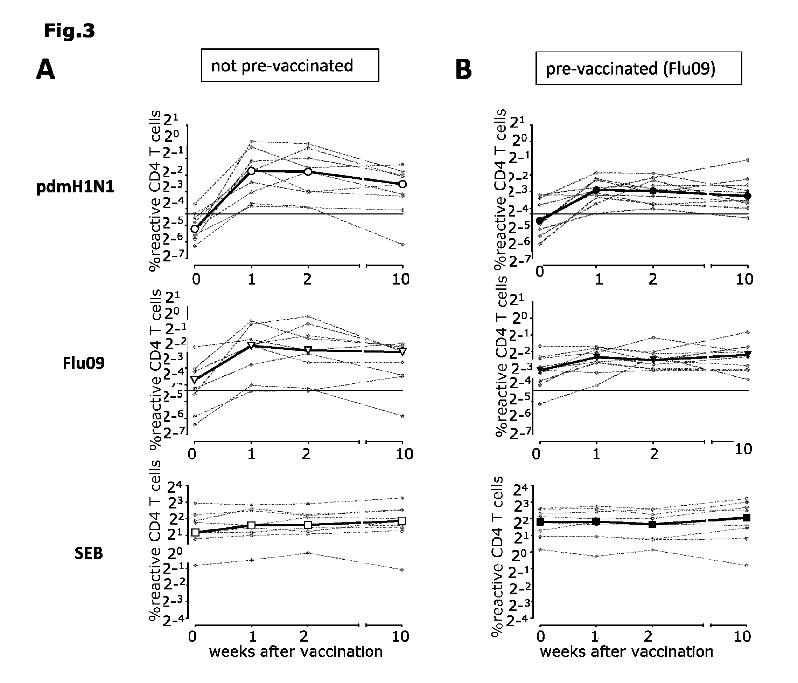

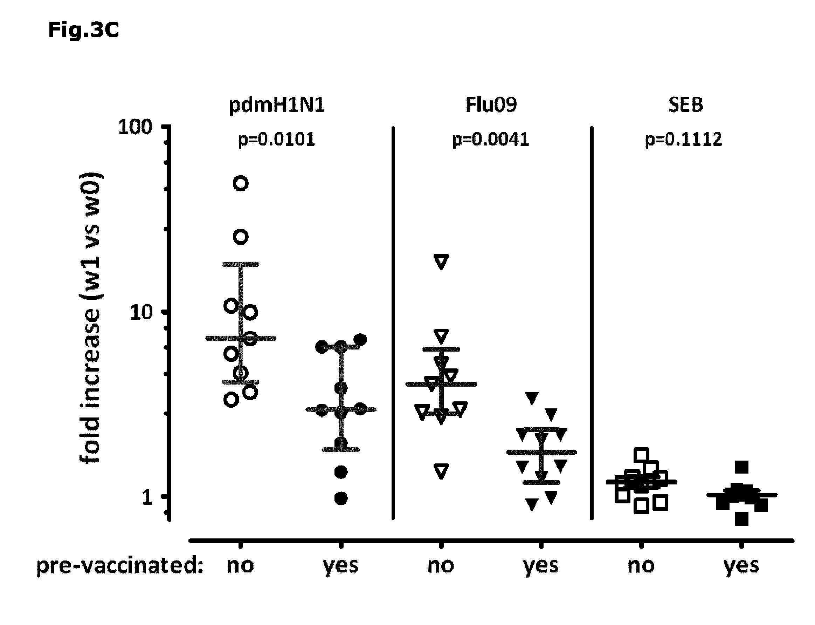

Figure 3: A pre-existing immunity prevents strong induction of antigen specific CD4 T cells. Frequencies of reactive CD4 T cells specific for the pandemic H1N1 antigen (pdmH1N1), the seasonal influenza vaccine Begrivac (Flu09) and *Staphylococcus aureus* enterotoxin B (SEB, positive control) in individuals that have not been pre-vaccinated (A) and those that have been pre-vaccinated with the seasonal influenza vaccine (B) are depicted over time. The detection limit (0.05% IFN-γ-producing CD69+ CD4 T cells) is represented by a horizontal line. (C) The increase of CD4 T-cell frequencies specific for pdmH1N1, Flu09 and SEB during the first week after vaccination with the pandemic vaccine was expressed as a multiple of the basal frequencies obtained before vaccination. Tested individuals were subdivided according to pre-vaccination status (pre-vaccinated against seasonal influenza or not). T-cell frequencies of each individual as well as median values and IQR are shown.

The kinetics in influenza- and SEB-reactive CD4 T cells in both groups are shown in figure 3A and B. Interestingly, the median increase in pdmH1N1 specific T-cell frequencies was significantly stronger in individuals that had not been pre-vaccinated against seasonal influenza (7.15-fold), as compared with individuals that had been pre-vaccinated (2.94-fold, p=0.01, Fig. 3C). Of note, this difference between the two groups was also found for CD4 T cells reacting towards seasonal influenza antigens (4.03-fold versus 1.74-fold, p=0.0041). The frequencies of SEB reactive T cells did not increase in any of the groups (p=0.11, Fig. 3C). The results indicate that a pre-existing immunity is associated with a less pronounced induction of influenza specific CD4 T cells after vaccination with Pandemrix.

### Vaccine-induced antibodies show only minor cross-reactivity with seasonal antigens

To compare the effects of the Pandemrix vaccine on cellular and humoral responses, induction of influenza-specific antibodies was determined. Importantly, vaccination induced a significant continuous increase in pdmH1N1-specific IgG titers which peaked about two weeks post vaccination and remained almost constant over the following eight weeks.

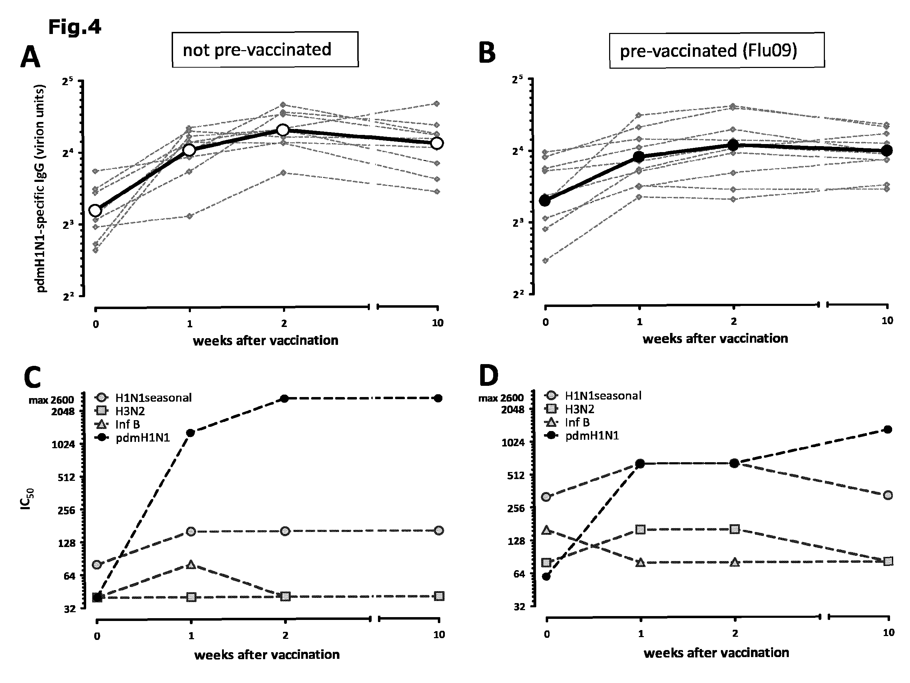

Figure 4: Vaccine-induced antibodies show only minor cross-reactivity with seasonal antigens. The induction of specific antibody responses towards pandemic H1N1 antigen was monitored by ELISA in individuals that have not been pre-vaccinated (A) and those that have been pre-vaccinated with the seasonal influenza vaccine (B). Serum samples of not pre-vaccinated (C) and pre-vaccinated individuals (D) were tested for virus-neutralising antibodies against pdmH1N1 and the single components of the seasonal Flu vaccine (H1N1seasonal, H3N2,

Influenza B (Inf B)). The IC50 value represents the respective serum dilution which leads to 50% virus neutralisation.

These kinetics were independent of pre-vaccination status (Fig. 4A and B). In order to assess potential cross-reactivity of antibodies induced by Pandemrix, plasma samples were analysed from individuals that were not pre-vaccinated against seasonal influenza for the presence of neutralising antibodies against pdmH1N1 and the individual compounds of the seasonal influenza vaccine (H1N1seasonal, H3N2, Influenza B), respectively. Consistent with results from ELISA (Fig. 4A), maximum titers of pdmH1N1-specific neutralising antibodies were induced already during the first two weeks following Pandemrix vaccination and remained stable throughout week 10 (Fig. 4C). In contrast, only a minor increase in seasonal H1N1 titers was observed indicating only slight neutralising cross-reactivity against the seasonal H1N1 influenza strain. Likewise, there was no significant effect on H3N2- and Influenza B-neutralising antibody titers. Individuals that had been pre-vaccinated against seasonal influenza had higher levels of neutralising antibodies towards the components of the seasonal vaccine that largely remained stable after Pandemrix vaccination (Fig. 4D). Interestingly, titers of pdmH1N1 neutralising antibodies before immunisation with the pandemic vaccine were also slightly elevated which may indicate a certain extent of cross-reactivity induced by the the seasonal vaccine. Of note, those titers showed an increase after Pandemrix vaccination, but did not reach the levels of those in individuals that had not been pre-vaccinated against seasonal influenza. Thus, these results demonstrate that vaccination with Pandemrix induces a pdmH1N1-specific antibody response with a certain extent of cross-reactivity towards seasonal H1N1 antigens. In line with cellular immunity, a pre-existing humoral immunity was associated with a less pronounced induction of pdmH1N1-specific titers after vaccination with Pandemrix.

Differences in the cytokine expression profiles of pdmH1N1-specific CD4 T cells discriminate vaccination responses from acute infection

Previous studies have indicated that acute viral or bacterial infections are associated with changes in cytokine expression profiles of antigen-specific T cells. To examine whether this is also the case in acute infection with pandemic H1N1 influenza virus, the IFN-γ/IL-2 expression profiles of specific T cells induced after vaccination were comparatively analysed with that of 7 patients that were suffering from a symptomatic infection with pandemic H1N1-influenza. Whole blood was stimulated with the pandemic and seasonal antigens as described before, and CD4 T cells were analysed flow-cytometrically for single or double expression of IFN-γ and IL-2. Stimulation with SEB served as positive control for cells with specificities unrelated to influenza.

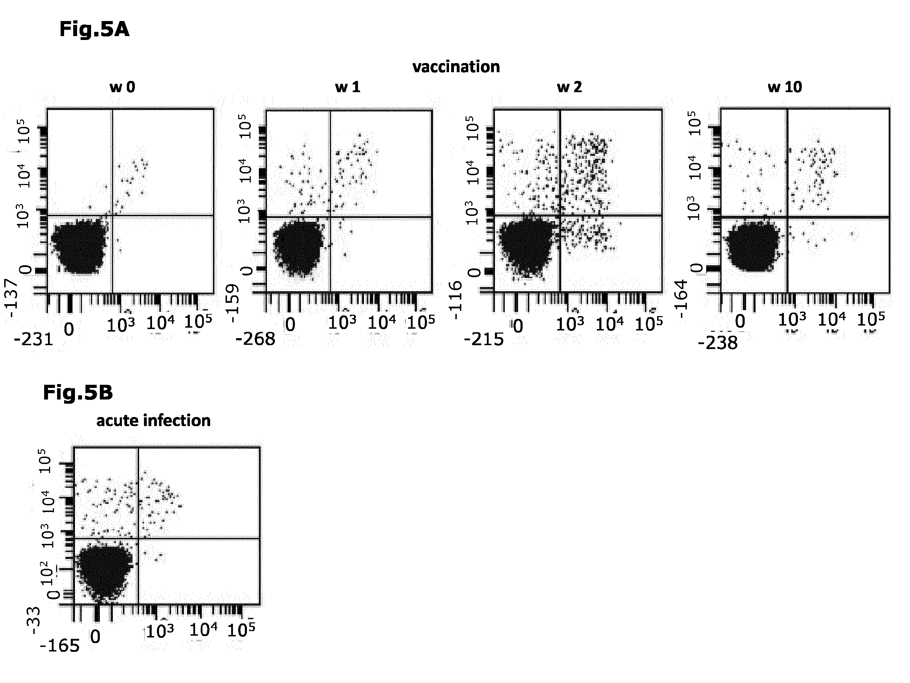

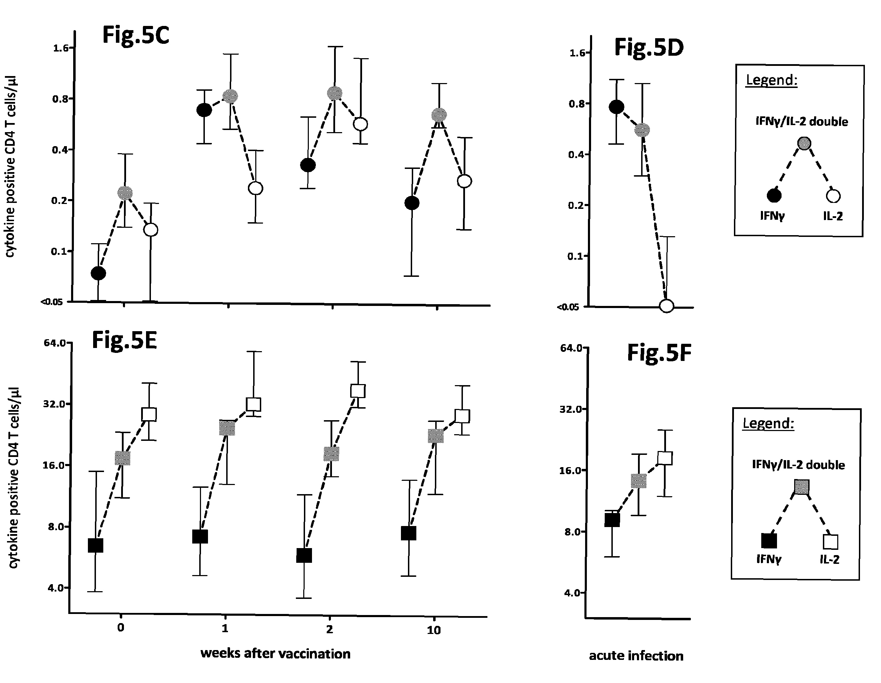

Figure 5: Differences in the cytokine expression profile of pdmH1N1-specific CD4 T cells discriminate memory or vaccination responses from acute infection. Representative dotplots of the flow cytometric analysis of IFN-γ and IL-2 expression in CD4 T cells after stimulation with pdmH1N1 antigen in blood samples of (A) one healthy individual (not pre-vaccinated against seasonal influenza) after vaccination with the pandemic influenza vaccine and (B) one patient with acute pandemic H1N1-influenza infection within one week after onset of symptoms. IFN-γ/IL-2 cytokine-profiling was performed in pdmH1N1 specific CD4 T cells of (C) all 19 individuals after immunisation with the pandemic influenza vaccine over time and (D) of 7 patients with acute pandemic H1N1 influenza infection. Shown are the median values and IQR of absolute numbers of IFN-γ single positive (black circles), IFN-γ/IL-2 double positive (grey circles) and IL-2 single positive (white circles) CD4 T cells per µl whole blood. Respective values after stimulation with SEB (positive control) are depicted for the vaccination cohort (E) and the infected patients (F).

Representative dotplots of influenza-specific IFN-γ/IL-2 cytokine profiles before and after vaccination and in an acutely infected patient are shown in figure 5A and B. Interestingly, the T-cell response in vaccinees was generally dominated by multifunctional CD4 T cells co-expressing both IFN-γ and IL-2 whilst single-cytokine producing cells were less abundant (Fig. 5C). Among the latter, those exclusively expressing IL-2 generally dominated over those producing IFN-γ only. This ratio changed only one week after vaccination. Thereafter, the profile largely normalised to that found prior to vaccination (Fig. 5C). As shown in figure 5D, this cytokine profile was markedly different from that found in patients with acute infections, where IFN-γ only expressing cells were most abundant and IL-2 single producing cells were completely absent. Of note, these profiles are indeed influenza-specific, as the cytokine profiles of SEB-reactive CD4 T cells were unaffected by vaccination (Fig. 5E) or acute infection (Fig. 5F). These results suggest that cytokine profiling of H1N1-specific CD4 T cells may allow distinction between vaccination responses and acute infection with the pandemic H1N1 influenza. Table

**Table 1: Kinetics of antigen-specific CD4 T-cell frequencies prior to and after immunisation with the pandemic influenza vaccine and fold increase during the first week following vaccination in all individuals (n=19).**

| | | pdmH1N1 | Flu09 | SEB |
|---|---|---|---|---|
| Median frequencies Basal (0 weeks) % (IQR) | | 0.03% (0.04%) | 0.10% (0.07%) | 3.40% (2.53%) |
| | 1 week | 0.18% (0.18%) | 0.24% (0.18%) | 3.21% (3.34%) |
| | 2 weeks | 0.14% (0.19%) | 0.21% (0.19%) | 3.13% (2.45%) |
| | 10 weeks | 0.12% (0.14%) | 0.24% (0.18%) | 4.01% (3.05%) |
| Median fold increase Week 1 vs. basal in specific T cells (IQR) | | 4.63 (4.16) | 2.71 (2.26) | 1.03 (0.25) |

| | | | | |
|---|---|---|---|---|
| Shown are median (IQR) frequencies of antigen-specific CD4 T cells (CD69+ IFN-γ+) that were calculated from values subtracted for the respective negative controls. | | | | |

### References

1. Sester, U., et al., PD-1 expression and IL-2 loss of cytomegalovirus- specific T cells correlates with viremia and reversible functional anergy. Am J Transplant, 2008. 8(7): p. 1486-97.
2. Zielinska, E., et al., Development of an improved microneutralization assay for respiratory syncytial virus by automated plaque counting using imaging analysis. Virol J, 2005. 2: p. 84.
3. Rowe, T., et al., Detection of antibody to avian influenza A (H5N1) virus in human serum by using a combination of serologic assays. J Clin Microbiol, 1999. 37(4): p. 937-43.

## Claims

1. An in-vitro process for the quick determination of the infection status of an infection with an influenza virus type from whole blood in terms of an acute influenza infection or a pre-immunization after previous, overcome influenza infection or induced immune response due to vaccination, comprising the steps of:
• stimulating an antigen-specific T cell that is present in a first sample of whole blood with antigens of the influenza virus type, optionally in the presence of antibodies against CD28, or CD28 and CD49d;
• processing of influenza antigens by antigen-presenting cells (APC) by incubation for a sufficient time, in particular by incubation of at least 0,5 h, particularly of 1.5 h to 2.5 h, especially for 2 h, at 35 °C to 39 °C, especially at 37 °C, optionally with adding CO₂;
• then adding a secretion inhibitor;
• effecting an intensive mixing;
• followed by a second incubation step for a period of at least 1 h, in particular 2.5 h, at a temperature of from 35 °C to 39 °C, and
• determining a profile from both the intracellular IFN-γ production and the intracellular IL-2 production of the antigen-specific T cell; wherein
• the presence of an acute influenza infection is indicated by a shift of the profile towards IFN-γ single positive cells accompanied by a ratio of IFN-γ/IL-2 single positive T cells larger than 1 compared to the ratio of IFN-γ/IL-2 single positive T cells of patients successfully vaccinated against, pre-infected or non-infected by the influenza virus which is smaller than 1.

2. The process according to claim 1, wherein a negative control is performed by adding a physiological buffer such as PBS 0.9% NaCl or 5% Glucose to an antigen-specific T cell that is present in the whole blood of a second sample, which is authentic with said first sample.

3. The process according to at least one of claims 1 or 2, wherein a positive control is performed by adding SEB to an antigen-specific T cell that is present in the whole blood of a third sample, which is authentic with said first sample.

4. The process according to at least one of the claims 1 to 3 wherein a vaccination control took place by adding the vaccination antigen to an antigen-specific T-cell that is present in the whole blood of a fourth sample which is authentic with said first sample.

5. The process according to at least one of claims 1 to 4, wherein said secretion inhibitor is selected from the group consisting of brefeldin A, monensin or the like.

6. The process according to at least one of claims 1 to 5, wherein the determination of the profile is effected by flow cytometry, automated microscopy or multiplex elisa methods.

7. The process according to at least one of claims 1 to 6, wherein the whole blood of the samples is derived from humans or animals.

## Patentansprüche

1. In-vitro-Verfahren zur schnellen Bestimmung des Infektionsstatus einer Infektion mit einem Influenzavirustyp aus Vollblut bezüglich einer akuten Influenzainfektion oder einer Vorimmunisierung nach vorangegangener überwundener Influenzainfektion oder einer induzierten Immunantwort aufgrund einer Impfung, umfassend die Schritte:
• Stimulieren einer antigenspezifischen T-Zelle, die in einer ersten Vollblutprobe vorhanden ist, mit Antigenen des Influenzavirustyps, gegebenenfalls in Gegenwart von Antikörpern gegen CD28 oder gegen CD28 und CD49d;
• Prozessieren von Influenza-Antigenen durch antigenpräsentierende Zellen (APC) durch Inkubation während einer ausreichenden Zeit, insbesondere durch Inkubation während wenigstens 0,5 h, insbesondere 1,5 h bis 2,5 h, insbesondere 2 h, bei 35 °C bis 39 °C, insbesondere 37 °C, gegebenenfalls unter Hinzufügen von CO₂;
• dann Hinzufügen eines Sekretionsinhibitors;
• Durchführen eines intensiven Mischens;
• gefolgt von einem zweiten Inkubationsschritt während wenigstens 1 h, insbesondere 2,5 h, bei einer Temperatur von 35 °C bis 39 °C; und
• Bestimmen eines Profils sowohl der intrazellulären IFN-γ-Produktion als auch der intrazellulären IL-2-Produktion der antigenspezifischen T-Zelle; wobei
• das Vorliegen einer akuten Influenzainfektion durch eine Verschiebung des Profils hin zu IFN-γ-einfach-positiven Zellen angezeigt wird, die einhergeht mit einem Verhältnis von IFN-y/IL-2-einfach-positiven T-Zellen von mehr als 1 im Vergleich zu dem Verhältnis von IFN-γ/IL-2-einfach-positiven T-Zellen von Patienten, die erfolgreich gegen das Influenzavirus geimpft wurden, mit diesem vorinfiziert oder nicht infiziert sind, das kleiner als 1 ist.

2. Verfahren gemäß Anspruch 1, wobei eine negative Kontrolle durchgeführt wird, indem man einen physiologischen Puffer, wie PBS mit 0,9% NaCl oder 5% Glucose, zu einer antigenspezifischen T-Zelle gibt, die im Vollblut einer zweiten Probe, die mit der ersten Probe authentisch ist, vorhanden ist.

3. Verfahren gemäß wenigstens einem der Ansprüche 1 oder 2, wobei eine positive Kontrolle durchgeführt wird, indem man SEB zu einer antigenspezifischen T-Zelle gibt, die im Vollblut einer dritten Probe, die mit der ersten Probe authentisch ist, vorhanden ist.

4. Verfahren gemäß wenigstens einem der Ansprüche 1 bis 3, wobei eine Impfkontrolle stattfand, indem man das Impfungsantigen zu einer antigenspezifischen T-Zelle gab, die im Vollblut einer vierten Probe, die mit der ersten Probe authentisch ist, vorhanden ist.

5. Verfahren gemäß wenigstens einem der Ansprüche 1 bis 4, wobei der Sekretionsinhibitor aus der Gruppe ausgewählt ist, die aus Brefeldin A, Monensin oder dergleichen besteht.

6. Verfahren gemäß wenigstens einem der Ansprüche 1 bis 5, wobei die Bestimmung des Profils mittels Durchflusscytometrie, automatischer Mikroskopie oder Multiplex-ELISA-Methoden erfolgt.

7. Verfahren gemäß wenigstens einem der Ansprüche 1 bis 6, wobei das Vollblut der Proben von Menschen oder Tieren stammt.

## Revendications

1. Procédé *in vitro* de détermination rapide du statut d'infection d'une infection avec un type de virus de la grippe à partir de sang total en termes d'une infection grippale aiguë ou d'une préimmunisation après une infection grippale antérieure maîtrisée ou une réponse immunitaire induite due à une vaccination, comprenant les étapes de :
• stimulation d'une cellule T spécifique de l'antigène qui est présente dans un premier échantillon de sang total avec des antigènes du type de virus de la grippe, facultativement en présence d'anticorps contre CD28, ou CD28 et CD49d ;
• traitement d'antigènes de la grippe par des cellules présentant l'antigène (CPA) par incubation pendant une durée suffisante, en particulier par incubation d'au moins 0,5 h, en particulier de 1,5 h à 2,5 h, spécialement pendant 2 h, à 35 °C à 39 °C, spécialement à 37 °C, facultativement avec ajout de CO₂;
• puis ajout d'un inhibiteur de sécrétion ;
• réalisation d'un mélange intensif ;
• suivie d'une seconde étape d'incubation pendant une période d'au moins 1 h, en particulier 2,5 h, à une température de 35 °C à 39 °C, et
• détermination d'un profil à partir à la fois de la production d'IFN-γ intracellulaire et de la production d'IL-2 intracellulaire de la cellule T spécifique de l'antigène ; dans lequel
• la présence d'une infection grippale aiguë est indiquée par un déplacement du profil vers des cellules T uniquement positives à IFN-γ accompagné d'un rapport de IFN-γ/IL-2 de cellules T uniquement positives supérieur à 1 comparé au rapport de IFN-γ/IL-2 de cellules T uniquement positives de patients vaccinés avec succès, préinfectés ou non infectés par le virus de la grippe qui est inférieur à 1.

2. Procédé selon la revendication 1, dans lequel un témoin négatif est réalisé par ajout d'un tampon physiologique tel que du PBS avec NaCl à 0,9 % ou du glucose à 5 % à une cellule T spécifique de l'antigène qui est présente dans le sang total d'un second échantillon, qui est authentique avec ledit premier échantillon.

3. Procédé selon au moins l'une des revendications 1 ou 2, dans lequel un témoin positif est réalisé par ajout de SEB à une cellule T spécifique de l'antigène qui est présente dans le sang total d'un troisième échantillon, qui est authentique avec ledit premier échantillon.

4. Procédé selon au moins l'une des revendications 1 à 3, dans lequel un témoin de vaccination a lieu par ajout de l'antigène de vaccination à une cellule T spécifique de l'antigène qui est présente dans le sang total d'un quatrième échantillon qui est authentique avec ledit premier échantillon.

5. Procédé selon au moins l'une des revendications 1 à 4, dans lequel ledit inhibiteur de sécrétion est choisi dans le groupe constitué par la bréfeldine A, la monensine ou similaire.

6. Procédé selon au moins l'une des revendications 1 à 5, dans lequel la détermination du profil est réalisée par une cytométrie en flux, une microscopie automatisée ou des méthodes ELISA multiplex.

7. Procédé selon au moins l'une des revendications 1 à 6, dans lequel le sang total des échantillons est dérivé d'humains ou d'animaux.
